# EUROPEAN PATENT APPLICATION

(11) **EP 1 211 326 A2**
(43) Date of publication of application: **05.06.2002**
(21) Application number: 01204552.2
(22) Date of filing: 28.11.2001
(51) Int. Cl.: C12Q 1/70

(54) **Diagnostic method**

(30) Priority: 30.11.2000 GB 0029270
(71) Applicant: GLAXO GROUP LIMITED, Greenford, Middlesex UB6 ONN (GB)
(72) Inventor: Blair, Edward Duncan, c/o GlaxoSmithKline, Stevenage, Herts, SG1 2NY (GB); Snowden, Barbara Wendy, c/o GlaxoSmithKline, Stevenage, Herts, SG1 2NY (GB); Ward, Chantelle Louise, c/o GlaxoSmithKline, Stevenage, Herts, SG1 2NY (GB)
(74) Representative: Giddings, Peter John, Dr.

(57) **Abstract**

The present invention relates to a method for detecting one or more indicator(s) of the probable onset of a chronic obstructive pulmonary disease (COPD) exacerbation; primers for use in the method; a computer model using detection of one or more indicator(s) to the probable onset of a COPD exacerbation for patient management and the prophylactic or curative treatment of COPD exacerbation and to a prognostic and/or diagnostic test for use in the detection of one or more indicator(s) to the probable onset of a COPD exacerbation.

SEQUENCE LISTING

<110> Glaxo Group Limited

<120> Diagnostic Method

<130> PG4334

<140>
<141>

<150> GB 0029270.6
<151> 2000-11-30

<160> 2

<170> PatentIn Ver. 2.1

<210> 1
<211> 16
<212> DNA
<213> Artificial Sequence

<220>
<223> Description of Artificial Sequence: Primer

<400> 1
cggacaccca aagtag 16

<210> 2
<211> 16
<212> DNA
<213> Artificial Sequence

<220>
<223> Description of Artificial Sequence: Primer

<400> 2
gcacttctgt ttcccc 16

## Description

### FIELD OF THE INVENTION

The present invention relates to a method of detecting one or more indicator(s) of the probable onset of a chronic obstructive pulmonary disease (COPD) exacerbation; primers for use in the method; a computer model using detection of one or more indicator(s) of the probable onset of a COPD exacerbation for patient management and the prophylactic or curative treatment of COPD exacerbation and to a prognostic and/or diagnostic test for use in the detection of one or more indicator(s) of the probable onset of a COPD exacerbation.

### BACKGROUND TO THE INVENTION

COPD is a disease characterised by inflammation of the airways. It includes, but is not limited to bronchitis and emphysema. Symptoms include coughing, wheezing and the production of mucus and the degree of severity can, in part, be viewed in terms of the volume and colour of secretions.

Patients with moderate to severe COPD are prone to exacerbations, associated with acute deterioration in symptoms and lung function, which increases in frequency with increasing severity of COPD. The COPD exacerbations have major effects on health status, are associated with morbidity and mortality and are frequently associated with upper respiratory tract infections.

Exacerbations may be characterised by coughing, wheezing and the production of sputum and may be measured as a decrease in forced expiratory volume measured over one second (FEV₁).

Asthma - an airways disease with some similarities but also some key differences to COPD - is a consequence of muscular spasm in the bronchi and may be triggered by the bursting of alveolar mast cells in response to an antigen.

In chronic bronchitis the bronchial mucous membrane is hypotrophised with the consequence that the bronchial lumen is narrowed. This causes airflow obstruction resulting in wheezing leading to respiratory insufficiency and sometimes even respiratory failure.

Emphysema is characterised by a breakdown of the lung architecture and may be measured by the production of the enzymes elastase and tryptase.

Because of the damage done when an exacerbation takes place it is desirable to predict the likely onset of an exacerbation and initiate treatments which either prevent the exacerbation occurring and/or treat the symptoms at an early stage thereby reducing the damage caused by the exacerbation.

To do this effectively it is necessary to:
1. Know the aetiology behind the exacerbation;
2. Be able to detect the cause(s) or some other indicator along the path to exacerbation in order to initiate an appropriate treatment; and
3. Treat the patient to prevent the onset of the exacerbation or at least reduce the damage caused by the exacerbation.

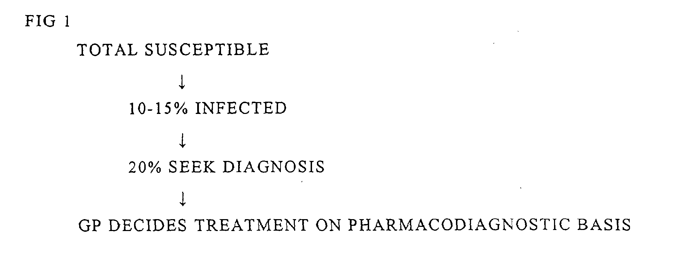

Viral infection has been associated with COPD but at present treatment is in response to the onset of exacerbation as determined by clinical symptoms. Thus, as illustrated in Fig 1, of the total susceptible population 10-15 % may be infected with something giving symptoms suggesting a COPD exacerbation is occurring. Of these about 20% may present themselves to a GP who will make a diagnosis and decide on the appropriate prescription, for example an antiviral or an antibiotic. As the GP is treating the symptoms, and not the cause which is often unknown, antibiotics are frequently over prescribed and this is a concern given the development of multi-resistant strains. It is thus desirable to understand the aetiology of COPD in order that preventative or prophylactic measures can be taken or, failing that, more precise treatment regimes prescribed.

Generally it has been observed that viral infections are more prevalent during the 'winter' months and a higher incidence of exacerbations occurs during the period September/October to December/January. The literature shows a widely varying percentage of exacerbations associated with virus (4.4 - 53%) with little agreement on the predominant viruses associated with exacerbation. Early studies suggested a predominance of influenza, but this has reduced in significance in recent studies.

However, literature interpretation is not straightforward due to variability in:
1. Definitions of both COPD and exacerbations;
2. The sensitivity of viral detection methods (e.g. culture conditions, insensitivity and the unreliability of serology for e.g. rhinovirus);
3. The sampling time after exacerbation onset;
4. The disease severity (variable guidelines); and
5. The lack of treatment / vaccination information.

One study, Journal of Clinical Microbiology Jan 1993 p.111-117, used PCR to demonstrate the presence of Picornavirus in subjects with and without respiratory symptoms. The study found that Picornavirus genomic material was found in 50% of symptomatic patients whereas they were isolated in only 16 % of the samples cultured.

Another study, AJRCCM 2000 161 (5): 1608 suggests rhinoviruses can be detected at COPD exacerbation and are associated with elevation of lower airway IL-6 that may mediate lower airway symptom expression during COPD exacerbations. In the study rhinoviruses were associated with 23% of COPD exacerbations. Detection was primarily in induced sputum sample and not nasopharyngeal sample suggesting rhinovirus directly infects the lower airway. There is no suggestion however that infection may precede COPD exacerbations.

It is an aim of the present invention to identify early indicators of (COPD) exacerbation and develop suitable predictive tests and treatment protocols based on these findings.

### SUMMARY OF THE INVENTION

According to a first aspect of the present invention there is provided a method, for predicting the likelihood of a patient suffering an onset of a chronic obstructive pulmonary disease (COPD) exacerbation, comprising: Assaying a patient sample for the presence of a virus and predicting the likelihood of the patient suffering an onset of a COPD exacerbation from said assay result.

According to a second aspect of the present invention there is provided a method, for predicting the likelihood of a patient suffering an onset of a chronic obstructive pulmonary disease (COPD) exacerbation, comprising: Assaying a patient sample for the presence of a rhinovirus and predicting the likelihood of the patient suffering an onset of a COPD exacerbation from said assay result.

According to a third aspect of the present invention there is provided a method, for predicting the likelihood of a patient suffering an onset of a chronic obstructive pulmonary disease (COPD) exacerbation, comprising: Assaying a patient sample for the presence of a virus and a second COPD exacerbation indicator; and predicting the likelihood of the patient suffering an onset of a COPD exacerbation from said assay result.

The second COPD exacerbation indicator may be an independent indicator, e.g. the presence or absence of a cytokine or an indicator related to the first indicator, e.g. a quantitive amount of said virus.

The term COPD as used herein is as defined by the Standards of Care Committee of the British Thoracic Society. It is characterised by airways obstruction which does not change markedly over several months and can be measured as a FEV₁ of less than 80 % of a normal predicted FEV₁.

Infact,
Mild COPD may be defined in terms of a FEV₁ of from 60-80% of the predicted norm;
Moderate COPD may be defined in terms of a FEV₁ of from 40-59% of the predicted norm; and
Severe COPD may be defined in terms of a FEV₁ of less than 40% of the predicted norm.

According to yet further aspects of the present invention there are provided diagnostic and / or prognostic methods and test kits for use in the detection of one or more indicator(s) to the probable onset of a COPD exacerbation and a computer model using detection of one or more indicator(s) of the probable onset of a COPD exacerbation for patient management and the prophylactic or curative treatment of COPD exacerbations.

Preferably the virus which is assayed for is a Picornavirus, more preferably still a rhinovirus.

Preferably the assay to determine the presence of a virus is a quantitive assay, more preferably a fluorogenic real time PCR assay or an assay detecting the presence of an antigen at a given load.

Where the assay detects rhinovirus it preferably employs primers having the sequences: 
Sequence ID no 1
5' CGG ACA CCC AAA GTA G 3';
 and

Sequence ID no 2
5' GCA CT T CTG TTT CCC C 3'

Preferably the quantitive assay measures viral nucleic acid and more particularly the copy number per volume of sample. The copy number may be used as a predictor of the likelihood of an exacerbation occurring and may thus be used to determine an appropriate treatment regime. In the case of rhinovirus a mean viral load of >5.9 x 10² could serve as a predictor.

The measure may be a direct measure or a comparative measure in which the sample is compared with a base line measure which may be patient specific.

Alternatively, the assay may be of a type which has been calibrated to only detect the presence of the virus if it is present at a given quantitive level.

Thus, results of investigations conducted by the applicant to date indicate viral load may be proportional to the severity of or imminence of an exacerbation.

In one study of rhinovirus loads in which the degree of exacerbation was assessed by way of chest score, differences in rhinovirus copy number of several orders of magnitude were noted and these are set out below:
A copy number of below 5,000 equated to a chest score of 0,
A copy number of about 20,000 equated to a chest score of 1;
A copy number of about 40,000 equated to a chest score of 2; and
A copy number of about 60,000 equated to a chest score of 3.

The sample type could be important in making the analysis and it is preferred that the sample is a nasopharyngeal sample although other samples, e.g. sputum could be used.

In one embodiment, the second indicator is a quantitive amount of the first indicator. In a second embodiment, the second indicator is the presence or absence of a different COPD exacerbation indicator. This may be an indicator of a viral infection, such as, for example, a cytokine, or a marker of cellular inflammation. Preferred cytokines include interleukins, for example, IL-6 or IL-8 and interferons, for example, IFNy. Preferred markers of cellular inflammation could be RANTES, EPO, MPO, EPX, and sICAM1.

The use of the second COPD exacerbation indicator may provide useful additional information as to the likelihood or imminence of an onset of exacerbation and can be used to determine the selection of a particular treatment. Thus, for example, the presence of the second indicator may tell you something more about the significance of the first indicator. For example the presence of interleukins, produced in response to an infection, and which will therefore lag behind an increase in viral load, may be used to determine whether a high viral load is on an upward or downward phase of growth. This information could prove significant in selecting an appropriate and effective therapeutic response. Thus, for example, if the viral load is increasing it would be appropriate to use an antiviral agent but if the bodies own defence system has already taken care of the virus and the levels are clearly decreasing it may no longer be appropriate to use an antiviral drug. The results could also suggest the use of a particular combination therapy, for example, the combination of an antiviral and an immunomodulator such as for example: steroids, IL5, sICAM1, leukotriene antagonists, 5LO, CpG, etc.

### BRIEF DESCRIPTION OF THE DRAWINGS

Fig 1 is a flow diagram illustrating current prescriptive practice for COPD patients;
Fig 2 is a graph illustrating mean Picornavirus viral load (copies/ml) of nasal aspirate versus chest score for COPD patients;
Fig 3 is graph showing exacerbation frequency in a patient group;
Fig 4 is a graph showing the % exacerbations that are rhinovirus positive in a seasonality study;
Fig 5 is a patient profile showing rhinovirus load both pre, during and post exacerbation;
Fig 6 is another patient profile showing rhinovirus load both pre, during and post exacerbation; and
Fig 7 is a flow diagram illustrating a prescriptive protocol based on the present invention.

The invention will now be described, by way of example only, with reference to the following examples.

### DETAILED DESCRIPTION OF THE INVENTION

### EXAMPLE 1.

### INTRODUCTION

A fluorogenic real time PCR method was developed and used to quantify viral nucleic acid in clinical samples.

An ABI7700 (TaqMan™) was used to quantify respiratory syncytial virus, influenza, Picornaviruses including rhinovirus, parainfluenza III and I, and adenovirus. These assays were applied to nasal aspirate samples taken from a cohort of children who were at the time experiencing exacerbation of asthma-like symptoms. Using clinical material from a retrospective study, the aims were to compare the sensitivity and specificity of quantitative TaqMan™ PCR with conventional PCR. Because of the comparative nature of the study it was also possible to further determine whether the virus titre measured in the upper airway was different in those positive by real time PCR only compared with those positive by both methods. The relationship between viral load in the upper airway and severity of symptom score was investigated and further details are given below.

### SUBJECTS

Nasal aspirate specimens were obtained from a cohort of 117 children aged 9-11 years with a history of wheeze or persistent cough in the previous 12 months, followed over a 13 month period as part of a longitudinal community based study. The cohort recorded a.m. and p.m. peak expiratory flow (best of three blows) and daily respiratory symptom scores, where 1 was mild, 2 was moderate and 3 was severe symptoms, between October 1994 and November 1995. The symptoms scored were wheeze on waking or during the day, cough on waking or during the day, shortness of breath or chest tightness, or any of the above during the night.

Nasal aspirate (NA) samples were obtained in the child's home whenever they had a fall in peak flow by greater than 50 litres/min from the normal value for that child, or a raised respiratory symptom score. More detailed scores were also recorded. The "Chest" score, included the following symptoms: runny nose / sneezing, blocked nose, sore throat / hoarse voice, head / face ache, and aches / chill / fever.
There were 221 nasal aspirate samples available for real time PCR testing that had parallel test results generated by conventional PCR for respiratory viruses. The samples had been frozen and thawed, and had been stored at -80°C for more than four years.

### SAMPLE PROCESSING FOR PCR

Total RNA and viral DNA were isolated using a spin column based method. As viral RNA cannot be detected directly by PCR, copy DNA (cDNA) was made from RNA using random hexamer primers and MuLV reverse transcriptase. Reactions were completed on a thermal cycler. Viral DNA was detected directly by PCR. DNA or cDNA from each sample was added to a specific PCR reaction mix for each virus in a 96 well plate. Thermal cycling was then performed on the ABI7700 and real time data was collected. The data was analysed using Sequence Detection System software.

The nucleic acids were extracted from samples for conventional PCR analysis by proteinase K digestion, phenol chloroform extraction and ethanol precipitation. The cDNA was made using specific primers (see Specificity Comparison below) and thermal cycling was performed on a conventional thermal cycler. PCR products were analysed on an ethidium bromide stained agarose gel.

### CALCULATION OF VIRAL LOAD

The copy number of viral DNA or cDNA was determined for each virus using TaqMan™ analysis by including a serially diluted plasmid standard of known concentration containing the DNA sequence from which primers and probe had been designed. A series of plasmid constructs were prepared that contained a portion of viral genome unique to each virus, but conserved between isolates of the same virus. The viral genome copy number is determined from the PCR cycle number at which the PCR signal is first detectable.

### SPECIFICITY COMPARISON

The real time and conventional PCR assays for Picornavirus were designed using the same primers for both in order to make a realistic comparison of the assay specificity.
The primers were: 
Sequence ID no 1
5' CGG ACA CCC AAA GTA G 3';
 and 
Sequence ID no 2
5' GCA CT T CTG TTT CCC C 3'
 which are complementary to the antisense RNA at position 542 to 557 and 169-185 in the 5' non coding region of RV1b.

These particular sequences were found to be particularly good at detecting a range of rhinovirus due to the conserved nature of these sequences in different rhinovirus types.

The results are shown in table 1 below:

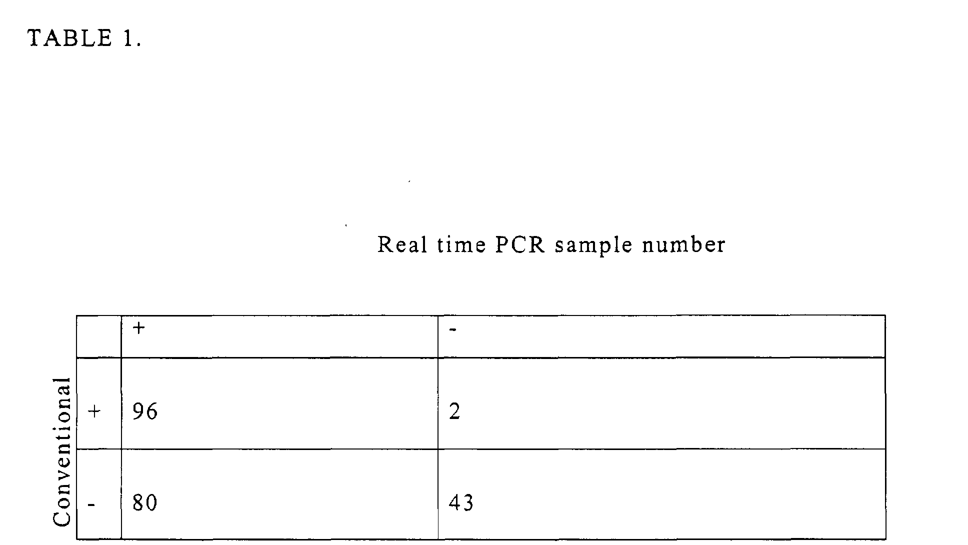

Ninety-eight percent (n=96) of positives detected by conventional PCR were also identified as positive by real time PCR.

Only 2% (n=2) of samples identified as positive by conventional PCR were negative by real time PCR.

Fifty-four percent (n=43) of those found to be negative by conventional PCR, were confirmed to be negative for Picornavirus by real time PCR.

The extra positives identified by real time PCR were a reflection of the difference in sensitivity between the two methods.

The assay gives a negative viral load reading when tested against other known stocks of viral cDNA.

### SENSITIVITY COMPARISON

Of those that were positive by real time PCR only (n=80) the median viral load was about 10,000 copies per ml of nasal aspirate.

Those positive by both methods had a median viral load of about 500,000 copies per ml. (p=<0.001, Mann-Whitney test)
The number of positive samples of different viral type detected by each technique is given in table 2 below.

**TABLE 2**

| Virus | Numbers positive from 221 tested by conventional PCR | Numbers positive from 221 tested by TaqMan PCR |
|---|---|---|
| Influenza | 7 | 7 |
| Picornavirus | 98 | 176 |
| Respiratory Syncytial Virus | 50 | 20* |
| Adenovirus | 1 | 18 |
| Parainfluenza I and III | 4 | 6 |

| | | |
|---|---|---|
| * Fewer positives were found due to the instability of the RSV virion over time whilst the samples were stored. | | |

### VIRAL LOAD AND CHEST SCORE

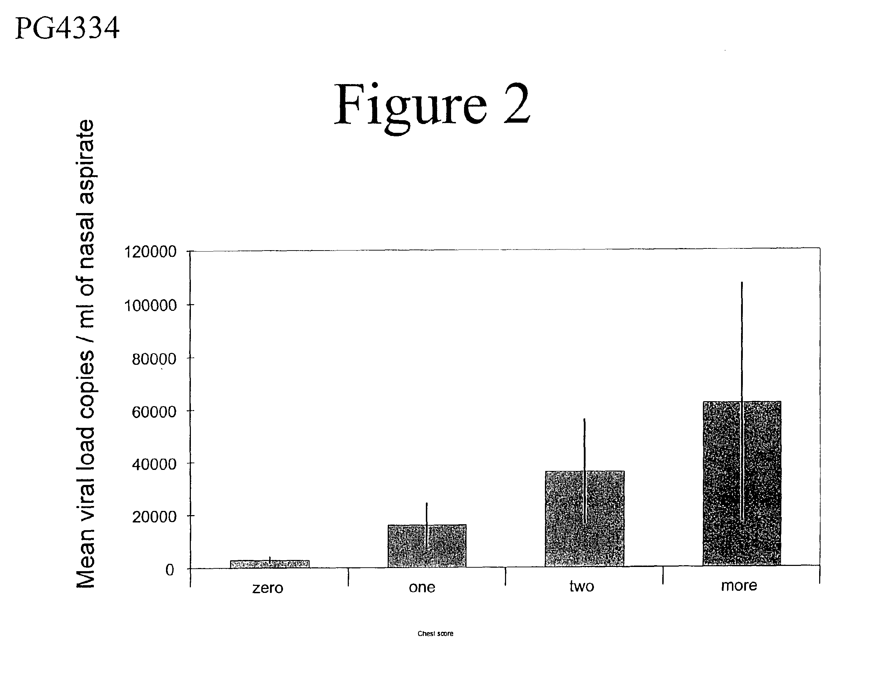

There was a trend of increased chest symptom severity and increased Picornavirus viral load in the upper airway as measured by real time PCR. The results are shown in Figure 2 which shows chest score severity versus viral load. (p)Mann-Whitney test for comparison of median viral loads of those with chest scores of zero or two).

### CONCLUSIONS

Fluorogenic real time quantitative PCR assays using TaqMan™ proved very effective at detecting respiratory viruses in clinical samples. The real time PCR assay was very sensitive for detecting rhinovirus positive samples. This real time PCR assay showed the presence of Picornavirus to be much more significant than previously thought with it showing itself to be active in 79.6% of patients who were symptomatic for COPD.

Interestingly, those viruses detected in samples by real time PCR but not by conventional PCR had lower mean viral loads compared with those that were detected by both methods suggesting the real time method is able to detect lower levels of virus. This could prove significant when looking to detect increases in viral load at an early stage to enable effective treatments, which are preventative rather than curative of exacerbations.

The results also indicate that there is a trend for increased viral load in the upper airway as chest symptom score increases which again suggest quantitive measurements have a significant role to play in prognostic and diagnostic tests and the management of COPD patients.

This study clearly shows a link between childhood asthma and respiratory viral infection, particularly Piconavirus, more particularly rhinovirus

### EXAMPLE 2.

In a further study the applicant undertook a viral load analysis of COPD clinical trial samples, Edinburgh (MacNee), to further study the association between virus and exacerbation.

The study was a single centre study in which 150 patients were studied for 2 years. Patients had 2 base line nasal swabs taken with additional samples taken on exacerbation (nasal swabs and spontaneous sputum).

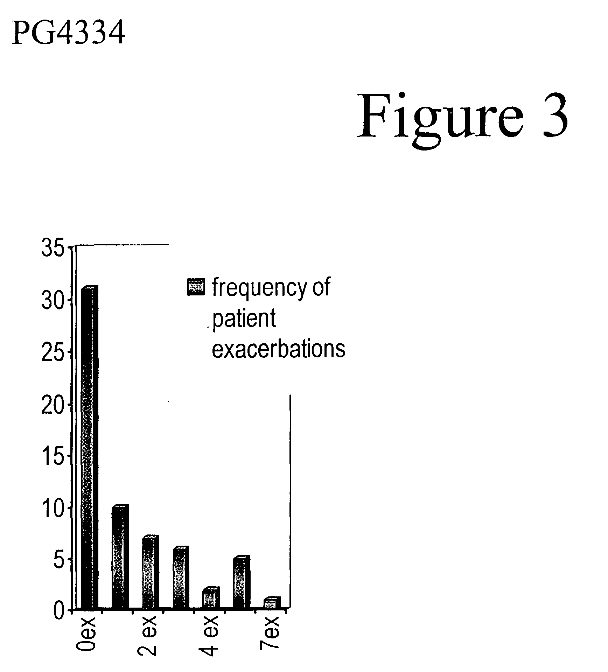

The frequency of exacerbation that patients experienced was noted. The results of the investigations are illustrated graphically in Fig 3.

The graph shows the number of exacerbations that patients experienced.125 samples were analysed from 59 COPD patients.58 were baseline samples and 67 were from exacerbation. The median age of the patients was 67 years (range 49-85 years).

The Percentage of virus positives at July 00 was as set out in table 3 below:

**TABLE 3**

| | EXACERBATIONS | BASELINE |
|---|---|---|
| NUMBER OF SAMPLES | 67 | 58 |
| NUMBER OF VIRUS POSITIVES | 15 | 3 |
| % VIRUS POSITIVE | 22 | 5 |

83.3% of virus positives were detected in exacerbation samples.

Viral loads from exacerbating patients were significantly different from baseline values (p = 0.0001)

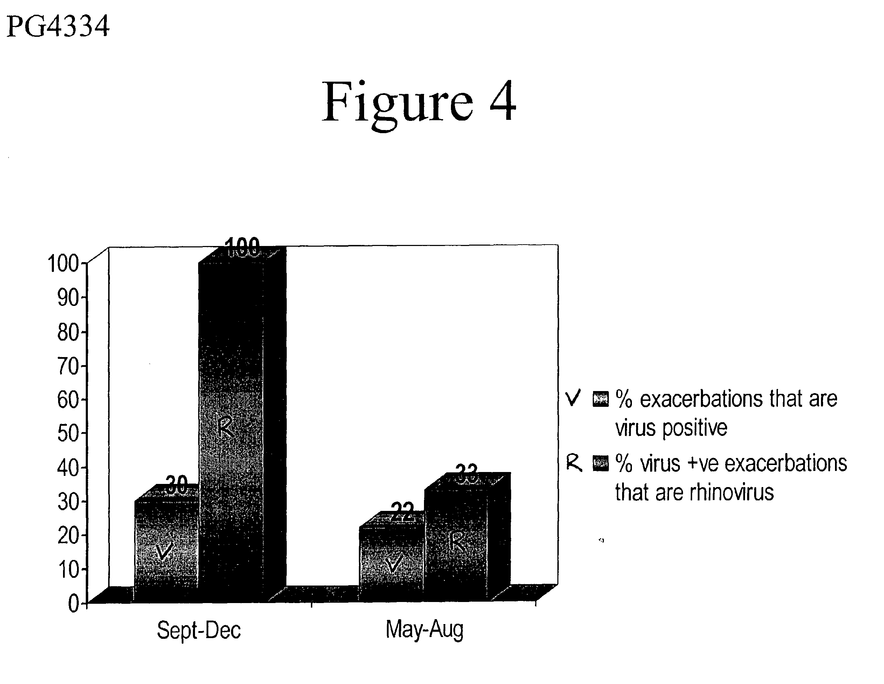

Significantly in the months September to December 100 % of virus positive exacerbations were rhinovirus. The results of the seasonality study are illustrated in Fig 4.

The virus positive exacerbations during these months are set out below:
- September to December 30%
- May to August 23%

The rhinovirus positive exacerbations during these months are set out below:
- September to December 100% of virus positive (i.e.30% of total exacerbations)
- May to August 33% of virus positive (i.e. 7.5% of total exacerbations)

Viral loads from exacerbating patients were significantly higher than baseline (p = 0.0001)

Viral load (copies / ml) was measured for all exacerbations and the results are shown in table 4 below:

**TABLE 4**

| VIRUS | BASELINE | | EXACERBATION | | |
|---|---|---|---|---|---|
| | NUMBER POSITIVE | MEAN VIRAL LOAD | NUMBER POSITIVE | MEAN VIRAL LOAD | % OF TOTAL VIRUS POSITIVE AT EXACEBATION |
| RHINOVIRUS | 0 | <5.9 x 10² | 10 | <1.1 x 10⁷ | 66.6 |
| CORONAVIRUS | 2 | 7.0 x 10³ | 2 | <1.8 x 10⁴ | 13.3 |
| PARAFLU III | 1 | 2.2 x 10⁴ | 2 | <3.0 x 10⁴ | 13.3 |
| INFLENZA A | 0 | <5.9 x 10² | 2 | <7.0 x 10⁵ | 13.3 |

### SUMMARY OF RESULTS:

Viral loads from exacerbating patients were significantly different from baseline values for both rhinovirus and influenza A (p = 0.0001)

The mean viral load for rhinovirus on exacerbation (n=15) is 7.1 x 10⁷ copies / ml, as compared with a mean viral load at baseline (n=3) of 1.1 x 10⁴ copies/ml.

Thus a test which detects a rise in copy number of 10ⁿ where ⁿ is at least 1, preferably at least 2 or 3, or measures quantitive loads of greater than a base line reading would significantly aid prognosis or diagnosis.

22% of exacerbations were virus positive and of these Picornavirus were the predominant virus. In fact, 100% of virus positive exacerbations that occur between September and December were rhinovirus. Clearly therefore rhinovirus is the most important target for any prognostic or diagnostic test / method.

If it could be demonstrated that such viral infection precedes COPD it would be possible to implement appropriate prognostic and diagnostic tests which could be used to effectively manage COPD patients and provide effective treatment regimes.

Thus the applicant proposed that a prognostic or diagnostic method which identified the presence of Piconavirus, e.g. rhinovirus, in a patient sample could form the basis of such a prognostic or diagnostic test and kit.

In order to determine whether rhinovirus infection precedes exacerbation a further study was undertaken, the further details of which are set out in Example 3.

### EXAMPLE 3.

### Lister study (Stanley).

A Cohort of 12 mild to severe COPD patients were followed both pre and post exacerbation.

The 12 patient study lasted for 12 weeks.

Nasal swabs were taken 3 times / week or daily when symptom scores and (FEV₁) indicated an exacerbation. Exacerbation was defined as 2 major or 1 major and 1 minor symptom for 2 consecutive days. Six out of twelve patients experienced exacerbation.

Patient 1 had 2 episodes.

Patients 3,4,6 & 8 had 1 episode.

Patient 10 had some symptoms for most of the study.

Of the 2 exacerbations analysed, both were Picornavirus positive.

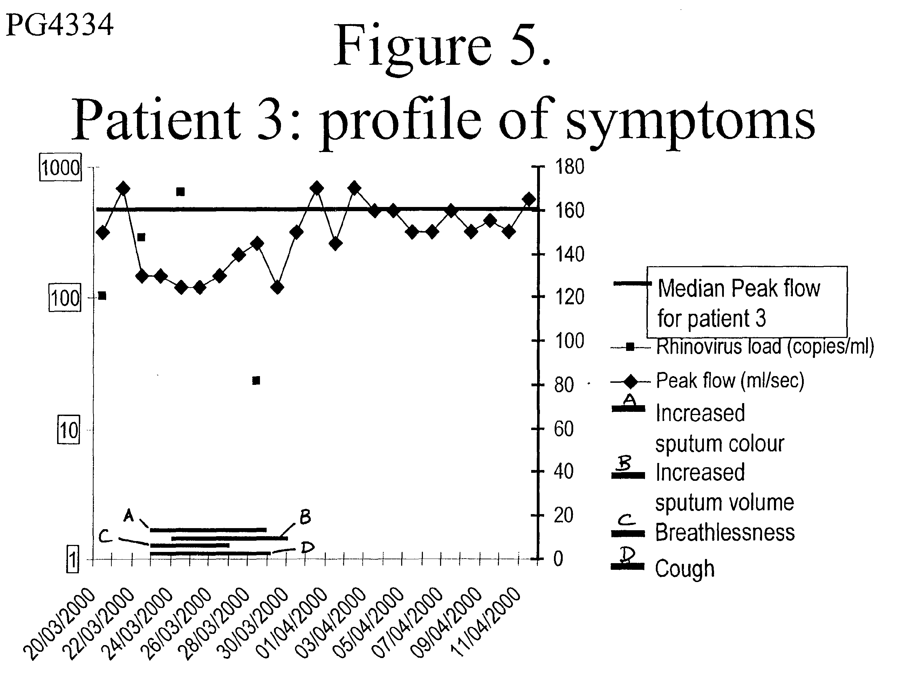

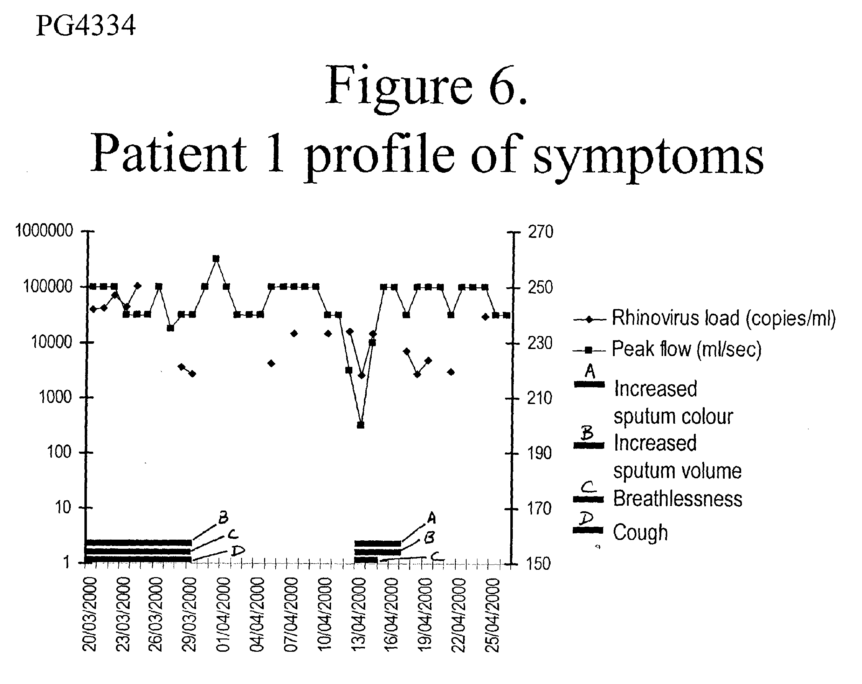

Their respective profiles are illustrated in Figs 5 and 6.

Fig 5 is the profile of patient 3. An increase in viral load can be seen to precede the exacerbation.

Fig 6 is the patient profile of patient 1 and again an increase in viral load can be seen to precede the exacerbations.

These findings are of particular significance in that they clearly demonstrate that by screening for rhinovirus and more particularly monitoring viral load changes it is possible to predict a likelihood of the onset of a COPD exacerbation in some patients.

### DEVELOPMENT OF PROGNOSTIC / DIAGNOSTIC / THERAPEUTIC PROTOCOL

From the results generated in examples 1, 2 and 3 above, and using, for example, the quantitive PCR method disclosed, it will be apparent that it is possible to produce diagnostic and prognostic methods (and kits) for screening for COPD exacerbation onset indicators, such as, for example, Picornavirus, more particularly rhinovirus. In addition, such methods and kits can be used in a patient management system for monitoring patients at risk from COPD exacerbations. Such a management system could include prescribing appropriate therapeutic agents in a timely manner, particularly combinations e.g. anti-virals and anti-inflammatories.

More particularly the method should measure viral load. This may be measured as for example a copy number, the number of infectious particles or plaque forming units (pfu) or some other measure which relates to viral load.

By screening for additional indicators, such as for example cytokines or indicators of infection, inflammation or airway hyperactivity e.g. elastase further information can be obtained making it possible to determine with more certainty whether particular treatments are likely to prove beneficial.

For example, the method could further comprise administering one or more therapeutic agents, such for example an anti-viral agent or a combination of agents such as for example an anti-viral in combination with an anti-inflammatory. Alternatively it might be determined that the exacerbation onset has arrived and that treatment with an anti-viral will no longer be the most appropriate therapy. In this instance the primary treatment could be an anti-inflammatory agent but adjunctive antiviral therapies may be appropriate given that immunomodular treatment could suppress host immune response to viral pathogens.

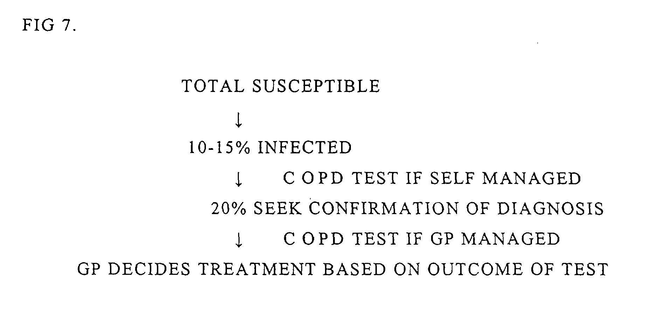

Thus a patient management system for the management of COPD patients might comprise an algorithm along the lines indicated in Fig 7 to predict the likelihood of a COPD exacerbation onset and the most appropriate treatment either prophylactic or curative based on the presence or absence of the indicators.

Thus the invention additionally comprises a system for diagnosing and or managing the onset of COPD exacerbation in a patient, which system comprises:
a means for testing the patient for at least one indicator of the onset of COPD exacerbation;
a means enabling the test results to be imputed into a data
management centre;
a means for analysing said test results; and
a means for generating a report including a prognosis and / or a recommended treatment.

In this regard a further problem to be overcome is to determine whether the patient has been diagnosed early enough for treatment to prevent exacerbation. The applicant has determined that the window of opportunity for treating effectively may be limited to a few days. Thus any prognostic or diagnostic method should ideally be able to differentiate between increasing and decreasing viral load.

One simple way of determining this would to be to test for the presence or absence of another indicator such as an immunogenic response marker or host inflammatory marker e.g. IL8 or EPO.

The results of the further test would also assist in determining whether combination therapies in which the pathogen is targeted with a drug, for example an antiviral and the symptoms are treated with other drug types such as for example anti-inflammatory. E.g. steroids or a respiratory drug such as for example Seretide/Advair, Flixotide or Serevent would be most appropriate. Another possible group of indicators, which could be targeted, would include cytokine modulatorsor soluble adhesion receptors.

In one embodiment a kit comprises a means for positively identifying rhinovirus. Preferably the means detects only a quantitively significant amount of virus indicative of the likely onset of a COPD exacerbation.

Preferably the kit is a rapid diagnostic (e.g. direct antigen) kit since such kits provides results in less than a few hours. Such rapid diagnostic kits include solid phase immuno assay kits, enzyme immuno assay membrane filter kits, monoclonal antibody based immuno assay kits and immuno fluorescent kits.

The kit will comprise each of the components enabling the sampling (e.g. taking of a nasopharyngeal swab) and running of the assay to geneerate a result, usually a colourmetric result.

## Claims

1. A method, for predicting the likelihood of a patient suffering an onset of a chronic obstructive pulmonary disease (COPD) exacerbation, comprising:
Assaying a patient sample for the presence of a virus and predicting the likelihood of the patient suffering an onset of a COPD exacerbation from said assay result.

2. A method as claimed in claim 1 wherein the virus is a Picornavirus.

3. A method as claimed in claim 2 wherein the Picornavirus is a rhinovirus.

4. A method as claimed in any of the preceding claims wherein the assay is a quantitive assay.

5. A method as claimed in any of the preceding claims wherein the assay is a fluorogenic real time PCR assay.

6. A method as claimed in claim 3 wherein the assay employs primers having the sequences: 
Sequence ID no 1
5' CGG ACA CCC AAA GTA G 3';
 and

Sequence ID no 2
5' GCA CTT CTG TTT CCC C 3'.

7. A method as claimed in any of the preceding claims wherein the assay measures an amount of viral nucleic acid (viral load) in the sample.

8. A method as claimed in claim 7 wherein a nucleic acid copy number per volume of sample is obtained.

9. A method as claimed in any of claims 7 or 8 wherein predicting the likelihood of the patient suffering from the COPD exacerbation involves comparing the viral load to a baseline figure.

10. A method as claimed in any of the preceding claims wherein when the assay detects rhinovirus, a viral load of >5.9 x 10² serves as a predictor of COPD exacerbation.

11. A method as claimed in any of the preceding claims wherein the sample is a nasopharyngeal sample

12. A method as claimed in any of the preceding claims wherein the method identifies a second COPD exacerbation indicator.

13. A method as claimed in Claim 12 wherein the second COPD exacerbation indicator is viral load.

14. A method as claimed in claim 12 wherein the second indicator is an indicator of a viral infection.

15. A method as claimed in claim 12 wherein the second indicator is a cytokine or a marker of cellular inflamation.

16. A method as claimed in claim 15 wherein the cytokine is an interleukin or an interferon.

17. A method as claimed in claim 16 wherein the interferon is IFNy.

18. A method as claimed in claim 16 wherein the interleukin is IL-6 or IL-8.

19. A method, for predicting the likelihood of a patient suffering an onset of a chronic obstructive pulmonary disease (COPD) exacerbation, comprising:
Assaying a patient sample for the presence of a rhinovirus and predicting the likelihood of the patient suffering an onset of a COPD exacerbation from said assay result.

20. A method, for predicting the likelihood of a patient suffering an onset of a chronic obstructive pulmonary disease (COPD) exacerbation, comprising:
Assaying a patient sample for the presence of a virus and a second COPD exacerbation indicator; and predicting the likelihood of the patient suffering an onset of a COPD exacerbation from said assay result.

21. A diagnostic or prognostic kit comprising means for detecting the presence of a virus and quantifying the amount present.

22. A system for diagnosing and / or managing the onset of a chronic obstructive pulmonary disease (COPD) exacerbation in a patient, which system comprises:
a means for testing the patient for at least one indicator of the onset of the COPD exacerbation;
a means enabling the test results to be imputed into a data management centre;
a means for analysing said test results; and
a means for generating a report including a prognosis and / or a recommended treatment.

23. A system as claimed in claim 22 further comprising prescribing a therapeutic agent.

24. A system as claimed in claim 22 wherein the prescribed therapeutic agent is an anti-viral.

25. A system as claimed in claim 22 wherein the prescribed therapeutic agent is an immunomodulator.

26. A system as claimed in claim 22 wherein the prescribed therapeutic agent is a plurality of agents.
